# EUROPÄISCHE PATENTANMELDUNG

(11) **EP 3 671 344 A1**
(43) Veröffentlichungstag der Anmeldung: **24.06.2020**
(21) Anmeldenummer: 18000986.2
(22) Anmeldetag: 20.12.2018
(51) Int. Cl.: G03F 7/00, G03F 7/027, G03F 7/029, G03F 7/039, G03F 7/20, A61B 5/00

(54) **VERFAHREN ZUR HERSTELLUNG EINES PHANTOMS FÜR BIOLOGISCHE SYSTEME**

(71) Anmelder: Technische Universität Wien, 1040 Wien (AT); Medizinische Universität Wien, 1090 Wien (AT)
(72) Erfinder: OVSIANIKOV, Aleksandr, A-1140 Wien (AT); GRUBER, Peter, A-1140 Wien (AT); WINDISCHBERGER, Christian, A-1170 Wien (AT)
(74) Vertreter: Keschmann, Marc

(57) **Zusammenfassung**

Bei einem Verfahren zur Herstellung eines Nervengewebsphantoms, das eine Vielzahl von Kanälen in einem Strukturmaterial aufweist, wobei die Kanäle vorzugsweise einen maximalen Querschnitt von 625 µm² aufweisen, wobei ein virtuelles Modell des Nervengewebsphantoms erstellt und das virtuelle Modell einer Strukturierungseinrichtung zugeführt wird, welche das Nervengewebsphantom mit Hilfe eines 3D-Druck-basierten, insbesondere lithographiebasierten, Strukturierungsverfahrens herstellt, ist das Strukturierungsverfahren als Multiphotonenlithographie-, insbesondere Multiphotonenabsorptionsverfahren ausgebildet, bei dem das einen Photosensibilisator oder Photoinitiator enthaltende Strukturmaterial ortsselektiv bestrahlt wird, wobei die Strahlung nacheinander auf Fokuspunkte innerhalb des Strukturmaterials fokussiert wird, wodurch jeweils ein am Fokuspunkt befindliches Volumenelement des Materials mittels einer photochemischen Reaktion in Folge von Multiphotonenabsorption eine Zustandsänderung erfährt.

## Beschreibung

Die Erfindung betrifft ein Verfahren zur Herstellung eines Phantoms für biologische Systeme, insbesondere Nervengewebe, das eine Vielzahl von Kanälen in einem Strukturmaterial aufweist.

Ein Nervengewebsphantom stellt eine Nachbildung eines Teilbereichs des menschlichen Nervengewebes dar. Mit Hilfe von Nervengewebsphantomen können Magnetresonanztomographen (MRT) effizienter kalibriert und Algorithmen zur besseren Unterscheidung der Axonverläufe implementiert werden. Die sichere Unterscheidung der Axonverläufe ist von wesentlicher Bedeutung, weil eine fehlerhafte Unterscheidung von einander kreuzenden, tangierenden und aneinander vorbeiführenden Axonen ein erhebliches Risiko beispielsweise bei der Operationsplanung in der Neurochirurgie darstellt.

Die Erfindung betrifft allgemein die Nachbildung jedes beliebigen Bereichs des menschlichen Nervengewebes. Bevorzugt bezieht sich die Erfindung auf die Nachbildung eines Teilbereichs des menschlichen Gehirns, wobei hier von einem Gehirnphantom gesprochen wird.

Bei der diffusionsgewichteten Magnetresonanztomografie (DW-MRI) handelt es sich um ein bildgebendes Verfahren, das mit Hilfe der Magnetresonanztomografie die Diffusionsbewegung von Wassermolekülen in Körpergewebe misst und räumlich aufgelöst darstellt. Bei der Diffusionstensorbildgebung (DTI) wird auch die Richtungsabhängigkeit der Diffusion erfasst. Innerhalb einer Probe bestimmt die Mikrostruktur des Materials die Beweglichkeit der Wassermoleküle und macht sie richtungsabhängig. Diese Richtungsabhängigkeit gibt Aufschluss über die Anisotropie und Mikrostruktur des Materials.

Eine interessante Anwendung von DTI ist die Untersuchung der weißen Substanz im Gehirn, die ein Netzwerk aus Bündeln paralleler Axonfasern enthält. In dieser Umgebung kommt es zur orientierungsabhängigen Diffusion, da die Diffusion entlang der Axonrichtung deutlich stärker als quer zur Faserrichtung ausgeprägt ist. Diese bevorzugte Diffusionsrichtung liefert Informationen über die Orientierung der Fasern. Die Zuverlässigkeit und Qualität der von der DTI erhaltenen Ergebnisse hängt jedoch von den erfassten Daten ab, die z.B. durch ein niedriges Signal-Rausch-Verhältnis, Patientenbewegungen während des Scans, chemische Verschiebung oder Inhomogenitäten des Magnetfelds oder eine geringe Auflösung beeinträchtigt sein können. Ferner ist die räumliche Auflösung der DTI um einige Größenordnungen niedriger als die Abmessungen der Fasern, und verschränkte oder einander kreuzende Fasern werden möglicherweise nicht richtig aufgelöst. Angesichts dieser Unsicherheiten ist es wünschenswert, die Genauigkeit der Ergebnisse und die gemessenen Diffusionsparameter und Faserrichtungen quantitativ zu überprüfen, um Fehlinterpretationen zu vermeiden. Dies kann durch direkte Messungen an Nervengewebsphantomen erreicht werden.

Nervengewebsphantome umfassen eine Vielzahl von Kanälen in einem Strukturmaterial, wobei die einzelnen Kanäle die Axonfasern nachbilden. Um die Bündel paralleler Axonfasern nachzubilden, muss ein Nervengewebsphantom dementsprechende Bündel paralleler Kanäle umfassen, wobei diese als einander kreuzende, tangierende und aneinander vorbeiführende Kanalbündel realisiert sein können. Um eine erfolgreiche Traktographie von an einem Nervengewebsphantom gewonnenen Diffusionstensorbildgebungsdaten betreiben zu können, sind sehr kleine Kanaldurchmesser unerlässlich. Ausgehend von der mittleren Diffusionslänge von Wassermolekülen bei Körpertemperatur von etwa 25 µm ergibt sich ein maximaler Kanaldurchmesser von etwa 20 µm. Gleichzeitig müssen die Kanalwände aber auch möglichst dünn sein, um den Gesamtanteil von Wassermolekülen und damit die erreichbare MR-Signalstärke zu maximieren. Zudem ist die hochaufgelöste Messung sehr kleiner Proben bzw. Nervengewebsphantome mit herkömmlichen Magnetresonanztomographen für die Humananwendung nicht möglich. Im Wesentlichen ist das Problem in der begrenzten Stärke der für die räumliche Kodierung notwendigen Magnetfeldgradienten begründet.

Die Herstellung von Nervengewebsphantomen mit einem eng angeordneten, offenporigen Kanalsystem erfordert somit ein Herstellungsverfahren, welches hochauflösende Mikrostrukturierungseigenschaften aufweist. Die Phantomstruktur soll sowohl Materialstabilität als auch einen klaren Diffusionsunterschied zwischen dem Strukturmaterial und den Kanälen aufweisen. Zudem soll gewährleistet sein, dass scharf begrenzte Kanäle gefertigt werden, da nur klar abgeschlossene und definierte Kanten am Übergang zu den Kanälen die punktgenaue Detektion von gerichteter Diffusion ermöglichen. Schließlich soll die Wirtschaftlichkeit des Verfahrens gegeben sein und die Herstellung daher mit einem ausreichenden Durchsatz möglich sein.

Die vorliegende Erfindung zielt darauf ab, ein Herstellungsverfahren für Nervengewebsphantome anzugeben, welches die oben genannten Anforderungen erfüllt.

Zur Lösung dieser Aufgabe sieht die Erfindung ein Verfahren zur Herstellung eines Nervengewebsphantoms vor, das eine Vielzahl von Kanälen in einem Strukturmaterial aufweist, wobei die Kanäle vorzugsweise einen maximalen Querschnitt von 625 µm² aufweisen, wobei ein virtuelles Modell des Nervengewebsphantoms erstellt und das virtuelle Modell einer Strukturierungseinrichtung zugeführt wird, welche das Nervengewebsphantom mit Hilfe eines 3D-Druck-basierten, insbesondere lithographiebasierten, Strukturierungsverfahrens herstellt, wobei das Strukturierungsverfahren als Multiphotonenlithographie-, insbesondere Multiphotonenabsorptionsverfahren ausgebildet ist, bei dem das einen Photosensibilisator bzw. Photoinitiator enthaltende Strukturmaterial ortsselektiv bestrahlt wird, wobei die Strahlung nacheinander auf Fokuspunkte innerhalb des Strukturmaterials fokussiert wird, wodurch jeweils ein am Fokuspunkt befindliches Volumenelement des Materials mittels einer photochemischen Reaktion in Folge von Multiphotonenabsorption eine Zustandsänderung erfährt.

Die Erfindung beruht somit auf der Erkenntnis, dass die Multi-Photonen-Absorption, insbesondere die Zwei-Photonen-Absorption (2PA) mit ihren hochauflösenden Mikrostrukturierungseigenschaften eine geeignete Methode darstellt, um Nervengewebsphantome zu erzeugen, welche die für die DTI-Kalibrierung erforderlichen Eigenschaften aufweisen. Mit 2PA können dauerhafte, langzeitstabile und kostengünstige Strukturen geschaffen werden.

Auf Grund der Nutzung von Multiphotonenabsorption (MPA) für die lithographiebasierte Fertigung des Nervengewebsphantoms können Bauteile mit hoher Auflösung bereitgestellt werden. Das verwendete Verfahren beruht darauf, dass die Zustandsveränderung des Materials nur in jenem Bereich des Strahlengangs stattfindet, in dem eine für MPA ausreichende Photonendichte vorliegt. Die höchste Photonendichte tritt im Brennpunkt des optischen Abbildungssystems auf, weshalb MPA nur im Fokuspunkt zu einer Zustandsveränderung des Materials führt. Durch eine solche inhärente Verkleinerung des aktiven Volumens ist die erreichbare Auflösung viel höher als bei herkömmlichen stereolithographiebasierten Verfahren und kann unter 100 Nanometern liegen.

Ein weiterer Vorteil von Multiphotonenabsorptionsverfahren liegt in der Möglichkeit, das Volumen des Fokuspunktes durch im Strahlengang angeordnete optische Einrichtungen den jeweiligen Bedürfnissen anzupassen, wodurch das Volumen des jeweiligen der Zustandsveränderung unterworfenen Volumenelements des Strukturmaterials in einfacher Weise eingestellt werden kann. Die Anpassbarkeit des Volumens der Volumenelemente ist besonders bei der Ausbildung von Kanalstrukturen, wie sie für Nervengewebsphantome charakteristisch sind, von Vorteil, wobei bevorzugt so vorgegangen werden kann, dass das Volumen an den zu erzielenden Kanalquerschnitt oder an den Kanalabstand zwischen benachbarten Kanälen angepasst wird. Insbesondere erfolgt dies derart, dass das Volumenelement bzw. dessen Querschnitt genau dem Kanalquerschnitt oder einem, vorzugsweise ganzzahligen, Bruchteil davon entspricht bzw. dass das Volumenelement bzw. dessen Erstreckung genau dem Kanalabstand oder einem ganzzahligen Bruchteil davon entspricht. Somit kann der Kanalquerschnitt oder das den Abstand zwischen benachbarten Kanälen ausfüllende Material durch ein einziges Volumenelement oder ein ganzzahliges Vielfaches davon strukturiert werden.

Gemäß einer bevorzugten Ausführung der Erfindung kann so vorgegangen werden, dass das Fokuspunktvolumen, nachdem es für die Herstellung eines Nervengewebsphantoms eingestellt wurde, für den gesamten Herstellungsprozess unverändert gelassen wird. Alternativ kann das Fokuspunktvolumen während des Verfahrens variiert werden, sodass das Nervengewebsphantom zustandsveränderte Volumenelemente einer ersten Gruppe und zustandsveränderte Volumenelemente einer zweiten Gruppe umfasst, wobei die volumenelemente der ersten Gruppe ein kleineres Volumen aufweisen als die Volumenelemente der zweiten Gruppe.

Das erfindungsgemäße Strukturierungsverfahren kann als additives oder als subtraktives Verfahren ausgebildet sein.

Im Falle eines additiven Verfahrens wird das jeweils im Fokuspunkt befindliche Volumenelement des Materials verfestigt. Es wird somit von einem Strukturmaterial ausgegangen, das im Ausgangszustand fließfähig ist und das während des Strukturierungsprozesses in einem Behälter gehalten wird, während die einzelnen Volumenelemente durch die Wirkung des auf den jeweiligen Fokuspunkt gerichteten Strahles verfestigt werden, sodass das Nervengewebsphantom aus den verfestigten Volumenelementen sukzessive aufgebaut wird. Der im Strukturmaterial enthaltene Photosensibilisator und/oder Photoinitiator ist hierbei ausgebildet, um im Strukturmaterial eine Photopolymerisation zu initiieren. Die Belichtung erfolgt bevorzugt von unten durch einen für die elektromagnetische Strahlung transparenten Boden des das fließfähige Material aufnehmenden Behälters.

Als Material zur Strukturierung mittels des additiven Multiphotonenabsorptionsverfahrens eignet sich bevorzugt wenigstens ein Acrylat- oder Methacrylat-basiertes Photopolymer, wie z.B. eine 1:1-Mischung von ethoxyliertem (20/3) - Trimethylolpropantriacrylat (ETA) und Trimethylolpropantriacrylat (TTA), vermischt mit einem Photoinitiator, wie z.B. M2CMK. Insbesondere kann eine Menge von 5 µmol M2CMK je Gramm der ETA-TTA-Mischung eingesetzt werden. Bei M2CMK handelt es sich um einen Stoff mit der folgenden molekularen Struktur:

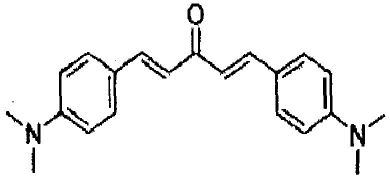

Im Zuge von Versuchen hat sich gezeigt, dass Nervengewebsphantome aus einer ETA-TTA-Mischung alle materialtechnischen Anforderungen erfüllen konnten. Die durchgeführten MR-Messungen zeigten deutliche Anisotropie in den Diffusionsmustern der Wassermoleküle und erlaubten dadurch erstmalig die erfolgreiche Anwendung der MRbasierten Traktographie in 3D-gedruckten Objekten.

Im Falle des subtraktiven Verfahrens wird das jeweils im Fokuspunkt befindliche Volumenelement des Materials photodegradiert. Es wird somit von einem im Ausgangszustand festen oder halbfesten Strukturmaterial ausgegangen, in dem die einzelnen Kanäle durch Photodegradation ausgebildet werden. Zur Ausbildung der Kanäle wird die Strahlung nacheinander auf Fokuspunkte innerhalb des Materials fokussiert, die im jeweils auszubildenden Kanal liegen, wodurch die an den Fokuspunkten befindlichen Volumenelemente des Materials photodegradiert werden. Aus der Gesamtheit von zusammenhängenden photodegradierten Volumenelementen entsteht nach dem Auswaschen des Photosensibilisators und zumindest eines Teils etwaiger Spaltprodukte jeweils ein Kanal.

Im Rahmen der Erfindung ist unter dem Begriff "photodegradieren" jeder Prozess zu verstehen, bei dem Materialbereiche auf Grund der Bestrahlung weiniger quervernetzt werden, ohne dass das Materialnetzwerk notwendiger vollständig degradiert wird. Es ist lediglich erforderlich, dass dadurch ein Bereich mit geänderten Diffusionseigenschaften entsteht, welcher Bereich im Sinne der Erfindung als "Kanal" zu verstehen ist.

Der im Strukturmaterial enthaltene Zweiphotonen-Photosensibilisator muss hierbei geeignet sein um eine photochemische Spaltung von in dem Strukturmaterial enthaltenden Bindungen zu bewirken. Materialien, in denen unter Zumischung von Zweiphotonen-Photosensibilisatoren und Anwendung einer elektromagnetischen Strahlung eine photochemische Spaltung von Bindungen bewirkt werden kann, sind aus dem Stand der Technik bekannt und können im Rahmen der vorliegenden Erfindung verwendet werden.

Eine besonders vorteilhafte Ausbildung sieht in diesem Zusammenhang vor, dass der Zweiphotonen-Photosensibilisator zum photochemischen Spalten von im Strukturmaterial enthaltenen photolabilen Bindungen, insbesondere Disulfidbindungen, ausgebildet ist, wobei der Photosensibilisator infolge der Zweiphotonen-Absorption, ohne eine intramolekulare Spaltung zu erfahren, die aufgenommene Energie auf die photolabilen Bindungen, insbesondere Disulfidbindungen überträgt und dadurch deren Spaltung bewirkt.

In einer bevorzugten Ausbildung enthält das Strukturmaterial die zu spaltenden photolabilen Bindungen, insbesondere Disulfidbindungen, in einer von einem Polymernetzwerk, vorzugsweise einem über diese Bindungen vernetzten Polymernetzwerk, gebildeten Matrix.

Das Polymernetzwerk ist dabei vorzugsweise ein Gel, vorzugsweise ein Hydrogel.

Die Vermischung des Zweiphotonen-Photosensibilisators mit dem Gel erfolgt gemäß weiterer bevorzugter Ausführungsformen der Erfindung auf einfache Weise, indem das Gel mit einer Lösung des Zweiphotonen-Photosensibilisators in einem geeigneten Lösungsmittel, bei Hydrogelen beispielsweise Wasser, quellen gelassen wird, um den Zweiphotonen-Photosensibilisator möglichst gleichmäßig im Gel zu verteilen, bevor die Bestrahlung erfolgt.

Eine solche Lösung des Zweiphotonen-Photosensibilisators weist vorzugsweise eine relativ niedrige Konzentration von nur etwa 0,01 mM bis etwa 1 mM, noch bevorzugter von etwa 0,05 mM bis etwa 0,5 mM, insbesondere von etwa 0,1 mM, auf, um Kosten zu sparen und ein Übermaß an Spaltungsreaktionen oder auch, in Abhängigkeit von der Zusammensetzung des Gels etwaige Nebenreaktionen zu verhindern.

Gemäß vorliegender Erfindung sind zwar prinzipiell alle bekannten Zweiphotonen-Photosensibilisatoren einsetzbar, allerdings wird vorzugsweise ein Photosensibilisator mit einem Zweiphotonen-Wirkungsquerschnitt σ2P > 1 GM (Goeppert-Mayer) im entsprechenden relevanten Spektralbereich eingesetzt, um keine übermäßig hohe Lichtintensität und/oder lange Bestrahlungsdauer wählen zu müssen.

In bevorzugten Ausführungsformen wird gemäß vorliegender Erfindung ein aus der Klasse der Benzylidenketone ausgewählter Zweiphotonen-Photosensibilisator, vorzugsweise einer der nachstehenden dargestellten Initiatoren P2CK, G2CK und E2CK, eingesetzt:

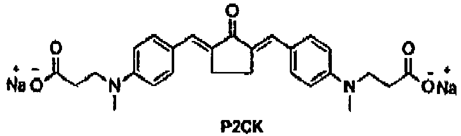

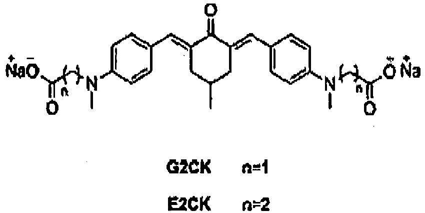

In anderen bevorzugten Ausführungsformen wird gemäß vorliegender Erfindung der nachstehend dargestellte Benzyliden-Initiator R1 eingesetzt:

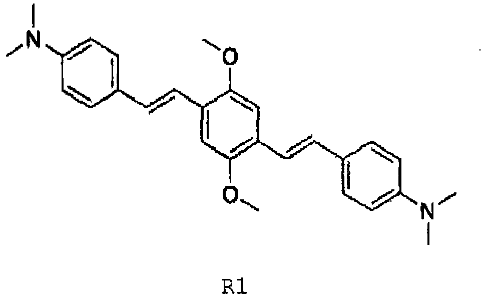

Das Prinzip der Multiphotonenabsorption beruht darauf, dass der photochemische Vorgang nur in jenen Bereichen des Strahlengangs stattfindet, in denen eine für die Multiphotonenabsorption ausreichende Photonendichte vorliegt. Die höchste Photonendichte tritt im Brennpunkt des optischen Abbildungssystems auf, sodass die Multiphotonenabsorption mit ausreichender Wahrscheinlichkeit nur im Fokuspunkt auftritt. Außerhalb des Brennpunktes ist die Photonendichte geringer, sodass die Wahrscheinlichkeit der Multiphotonenabsorption außerhalb des Brennpunktes zu gering ist, um eine unumkehrbare Veränderung des Materials durch eine photochemische Reaktion zu bewirken. Die elektromagnetische Strahlung kann in der verwendeten Wellenlänge weitestgehend ungehindert das Material passieren und nur im Fokuspunkt kommt es zu einer Interaktion zwischen photosensitivem Material und elektromagnetischer Strahlung. Das Prinzip der Multiphotonenabsorption ist beispielsweise in Zipfel et al, "Nonlinear magic: multiphoton microscopy in the biosciences", NATURE BIOTECHNOLOGY VOLUME 21 NUMBER 11 NOVEMBER 2003, beschrieben.

Als Quelle für die elektromagnetische Strahlung kann es sich vorzugsweise um einen kollimierten Laserstrahl handeln. Der Laser kann sowohl eine oder mehrere, feste oder variable Wellenlängen emittieren. Insbesondere handelt es sich um einen kontinuierlichen oder gepulsten Laser mit Pulslängen im Nanosekunden-, Pikosekunden- oder Femtosekunden-Bereich. Ein gepulster Femtosekundenlaser bietet dabei den Vorteil, dass eine geringere mittlere Leistung für die Multiphotonenabsorption benötigt wird.

Die Leistung der elektromagnetischen Strahlung und die Belichtungsdauer beeinflussen die Qualität des erzeugten Bauteils. Durch Anpassung der Strahlungsleistung und/oder der Belichtungsdauer kann das Volumen des Fokuspunktes in einem engen Bereich variiert werden. Bei zu hohen Strahlungsleistungen treten zusätzliche Prozesse auf, die zur Beschädigung des Bauteils führen können. Ist die Strahlungsleistung zu gering, kann sich keine dauerhafte Materialeigenschaftsänderung einstellen. Für jedes photosensitive Material gibt es daher typische Bauprozessparameter die mit guten Bauteileigenschaften verbunden sind.

Wie bereits erwähnt, kann das Fokuspunktvolumen bei einer bevorzugten Ausbildung der Erfindung während des Verfahrens derart variiert werden, dass das Nervengewebsphantom zustandsveränderte, insbesondere verfestigte oder photodegradierte, Volumenelemente einer ersten Gruppe und zustandsveränderte, insbesondere verfestigte oder photodegradierte, Volumenelemente einer zweiten Gruppe umfasst, wobei die Volumenelemente der ersten Gruppe ein kleineres Volumen aufweisen als die Volumenelemente der zweiten Gruppe. Die genannte Variation des Fokuspunktvolumens beruht hierbei jedoch nicht auf einer Änderung der Intensität der verwendeten elektromagnetischen Strahlung. Vielmehr wird bei der für den Herstellungsprozess gewählten (optimalen) Strahlungsintensität gearbeitet, die während des Prozesses mit Vorteil unverändert gelassen wird. Das erfindungsgemäße Verfahren wird daher bevorzugt so durchgeführt, dass die Änderung des Fokuspunktvolumens bei gleichbleibender Strahlungsintensität vorgenommen wird, wobei die verwendete durchschnittliche Leistung der elektromagnetischen Strahlung dementsprechend angepasst wird.

Als Fokuspunktvolumen wird daher das Volumen eines belichteten Punktes nach dem Aufbereitungsschritt bei den typischen Prozessparametern verstanden. Unter der Änderung des Fokuspunktvolumens wird eine Veränderung der räumlichen Intensitätsverteilung im Fokuspunkt verstanden. Hierbei kann die räumliche Intensitätsverteilung des Fokuspunktes sowohl in eine oder mehrere Richtungen verändert werden. So kann zum Beispiel durch Reduktion der effektiven numerischen Apertur des optischen Abbildungssystems die Intensitätsverteilung in alle drei Raumrichtungen vergrößert werden. Bei der Verwendung eines diffraktiven optischen Elementes kann der Fokus in eine Linie oder Fläche verändert, oder die Anzahl der Fokuspunkte erhöht werden.

Durch eine Variierung des Fokuspunktes während der Herstellung des Nervengewebsphantoms bleibt bei kleinem Fokuspunktvolumen der Vorteil der hohen Auflösung erhalten, während bei großem Fokuspunktvolumen eine hohe Schreibgeschwindigkeit (gemessen in mm³/h) erzielbar ist.

Bei einem additiven Herstellungsprozess kann die Variation des Fokuspunktvolumens so genutzt werden, dass die erste Gruppe von verfestigten Volumenelementen die Kanalwände ausbilden und die zweite Gruppe von verfestigten Volumenelementen die zwischen den Kanalwänden liegenden Bereiche ausbilden. Kleine Fokuspunktvolumina werden somit für die scharfe Begrenzung der Kanäle verwendet, wohingegen große Fokuspunktvolumina zum Füllen der dazwischen liegenden Innenräume und ggf. für die Randbereiche des Nervengewebsphantoms verwendet werden. Die Kanalwände, die aus den verfestigten Volumenelementen der ersten Gruppe bestehen, weisen hierbei zumindest die Dicke eines Volumenelements auf. Die Kanalwände können auch die Dicke von zwei, drei oder mehreren Volumenelementen aufweisen. Die Dicke der aus den verfestigten Volumenelementen der ersten Gruppe bestehenden Kanalwände ist jedoch derart begrenzt, dass zwischen den Kanalwänden benachbarter Kanäle noch ein Bereich verbleibt, der aus den verfestigten Volumenelementen der zweiten Gruppe gebildet ist.

Bei eine subtraktiven Herstellungsprozess kann die Variation des Fokuspunktvolumens so genutzt werden, dass die erste Gruppe von photodegradierten volumenelementen die Kanalwände ausbilden und die zweite Gruppe von photodegradierten Volumenelementen den Innenraum der Kanäle ausbilden. Kleine Fokuspunktvolumina werden somit für die scharfe Begrenzung der Kanäle verwendet, wohingegen große Fokuspunktvolumina zum Photodegradieren des von den Wänden begrenzten Innenraums verwendet werden.

Bei einer bevorzugten Verfahrensweise wird das Volumen des Fokuspunkts während des Verfahrens derart variiert, dass die zustandsveränderten Volumenelemente der ersten Gruppe ein Volumen aufweisen, das weniger als 50%, bevorzugt weniger als 10%, des Volumens der zustandsveränderten Volumenelemente der zweiten Gruppe beträgt.

Bevorzugt erfolgt die Variation des Fokusvolumens derart, dass das größte Fokuspunktvolumen während der Fertigung des Nervengewebsphantoms größer 50µm³, bevorzugt größer 100µm³, insbesondere größer 10.000µm³ ist.

Bevorzugt erfolgt die Variation des Fokusvolumens derart, dass das kleinste Fokuspunktvolumen während der Fertigung des Nervengewebsphantoms kleiner 50µm³, bevorzugt kleiner 1µm³, insbesondere kleiner 0,05µm³ ist.

Der Querschnitt der im Nervengewebsphantom ausgebildeten Kanäle kann den jeweiligen Bedürfnissen entsprechend gewählt werden. Bevorzugt ist der Querschnitt quadratisch, rechteckig, kreisrund oder oval.

Die Querschnittsfläche der Kanäle ist wie bereits erwähnt kleiner 625 µm². Bevorzugt wird die Querschnittsfläche kleiner 100 µm², insbesondere kleiner 50 µm² gewählt.

Um die bei einem menschlichen Gehirn typischerweise vorhandene Topographie der Bündel paralleler Axonfasern nachbilden zu können, sieht eine bevorzugte Ausbildung der Erfindung vor, dass das Nervengewebsphantom eine erste Gruppe von Kanälen und eine zweite Gruppe von Kanälen umfasst, wobei die Kanäle der ersten Gruppe und die Kanäle der zweiten Gruppe nicht parallel zueinander verlaufen. Dabei bildet die erste Gruppe von Kanälen ein erstes Kanalbündel und die zweite Gruppe von Kanälen bildet ein zweites Kanalbündel.

Insbesondere kreuzen die Kanäle der ersten Gruppe die Kanäle der zweiten Gruppe, vorzugsweise in einem rechten Winkel.

Weiters können das erste Kanalbündel und das zweite Kanalbündel tangierend ausgebildet sein. In diesem Zusammenhang ist bevorzugt vorgesehen, dass die Kanäle der ersten und der zweiten Gruppe gekrümmt ausgebildet sind und eine gemeinsame Tangente aufweisen.

Gemäß eines gesonderten Aspekt der vorliegenden Erfindung wird die Verwendung eines nach dem erfindungsgemäßen Verfahren hergestellten Nervengewebsphantoms zur Kalibrierung eines Magnetresonanztomographen beansprucht, wobei das Nervengewebsphantom einer diffusionsgewichteten MRT, insbesondere Diffusionstensorbildgebung und/oder Mehrschalen- bzw. q-Ball Bildgebung, unterworfen wird und die gewonnenen Bildgebungsdaten einer quantitativen Analyse und/oder einer Traktographie unterworfen werden, wobei das Ergebnis der quantitativenquantitativen Analyse mit den Diffusionsreferenzwerten verglichen wird, während das Traktographieergebnis mit dem tatsächlichen Verlauf der Kanäle im Nervengewebsphantom verglichen wird und etwaige Abweichungen zur Kalibrierung des Magnetresonanztomographen herangezogen werden. Es versteht sich, dass die Kanäle mit einem Kontrastmittel gefüllt werden können, bevor das Nervengewebsphantom der DiffusionsDiffusions-MRT unterworfen wird.

Die Erfindung wird nachfolgend anhand von in der Zeichnung schematisch dargestellten Ausführungsbeispielen näher erläutert. In dieser zeigen Fig. 1 eine Vorrichtung zur Durchführung des erfindungsgemäßen Verfahrens, Fig. 2 eine Einheit zur Fokusvolumenanpassung, Fig. 3 eine abgewandelte Ausbildung der Einheit zur Fokusvolumenanpassung, Fig. 4 eine weitere abgewandelte Ausbildung der Einheit zur Fokusvolumenanpassung, Fig. 5 eine weitere abgewandelte Ausbildung der Einheit zur Fokusvolumenanpassung, Fig. 6 eine weitere abgewandelte Ausbildung der Einheit zur Fokusvolumenanpassung und die Fig. 7a-c verschiedenen Ausführungen der Kanalstruktur in einem Nervengewebsphantom.

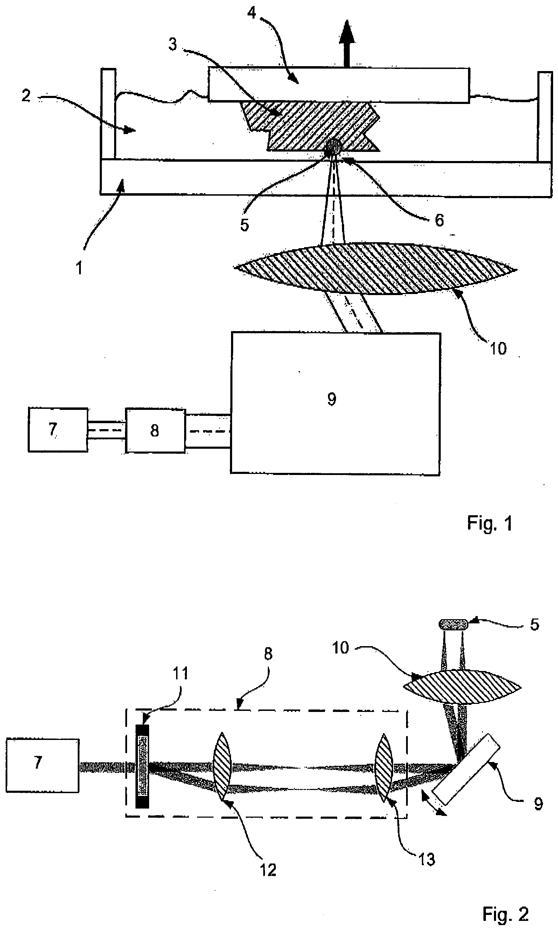

In Fig. 1 ist ersichtlich, dass eine vom Laser 7 emittierte elektromagnetische Welle durch eine Einheit zur Veränderung des Fokuspunktvolumens 8 und über eine Strahlablenkeinheit 9 geführt und mittels eines optischen Abbildungssystems 10 durch eine transparente Bodenplatte 1 in ein photosensitives Material 2 fokussiert wird. Im Brennpunkt 5 des optischen Abbildungssystems 10 kommt es zur Zustandsänderung des photosensitiven Materials 2, womit das Bauteil 3 aufgebaut wird. Das Bauteil 3 hängt an einer Bauplattform 4, die in vertikaler Richtung bewegt werden kann. Nachdem die aktuelle Schicht fertig belichtet wurde, wird die Bauplattform 4 angehoben und die nächste Schicht wird belichtet. Durch Verwendung eines Lasers 7 mit hoher Spitzenleistung ist der Einsatz von Multiphotonenabsorption möglich, sodass die Zustandsänderung des Materials nur im Fokus 5 stattfindet, nicht aber in der optisch "toten" Zone 6 zwischen dem Wannenboden 1 und der Bauplattform bzw. dem bereits gebildeten Bauteil. Somit kann es zu keiner Anhaftung des Bauteils an der transparenten Bodenplatte 1 kommen.

Sämtliche Elemente in Fig. 1 sind lediglich symbolisch dargestellt und können beliebig und entsprechend dem Wissen des Fachmanns erweitert werden, beispielsweise durch Einsatz zusätzlicher Linsensysteme, Blenden, Spiegel, Filter oder Strahlteiler.

Fig. 2 zeigt schematisch die Einheit 8 zur Veränderung des Fokuspunktvolumens. Die Einheit umfasst ein diffraktives optisches Element 11, welches den eintreffenden Strahl in zwei Strahlen aufspaltet, die ein System aus zwei Linsen 12 und 13 durchlaufen. Die Aufspaltung des Strahls erfolgt mit dem Ziel, zwei Punkte nebeneinander in der Fokusebene zu erzeugen. Überlappen einander beide Punkten, kann man auch von einer Linie sprechen. Alternativ kann das Element 11 als schnell bewegtes Strahlablenksystem ausgebildet sein, mit dem die Breite des Fokusvolumens einstellbar ist.

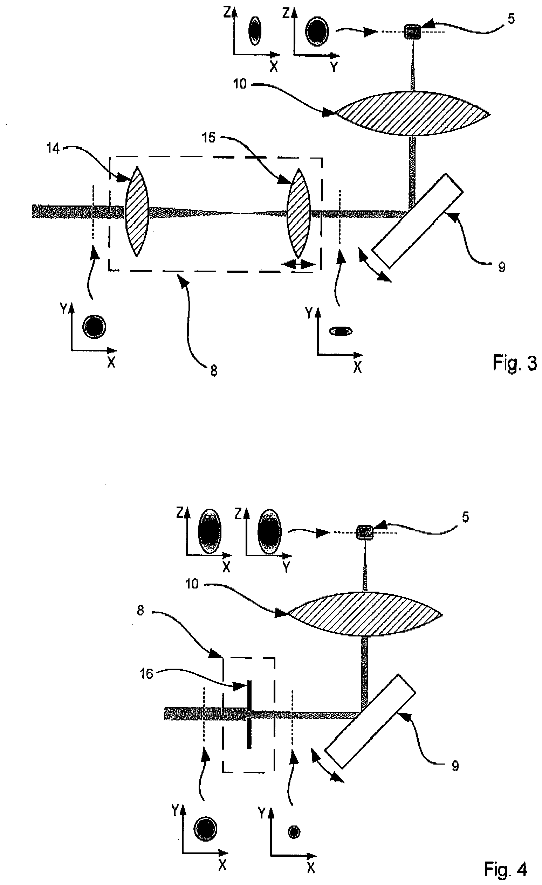

Fig. 3 zeigt eine abgewandelte Ausbildung der Einheit 8 zur Fokusvolumenanpassung. Die Einheit 8 umfasst zwei voneinander beabstandete koaxiale Zylinderlinsen 14 und 15, welche von dem Strahl durchlaufen werden. Das Strahlprofil vor dem Eintritt in die Einheit 8, und zwar in der strichliert dargestellten Ebene, ist kreisrund. Am Austritt aus der Einheit 8 ist eine Komprimierung in Richtung der y-Achse zu beobachten. Daraus ergibt sich am Fokuspunkt 5 in der x,z-Ebene und in der y,z-Ebene die in der Zeichnung dargestellt Ausdehnung des Fokuspunktvolumens.

Durch Einstellung des Abstands zwischen den Zylinderlinsen 14 und 15 ändert sich das Volumen des Gesamt-Fokuspunkts 5.

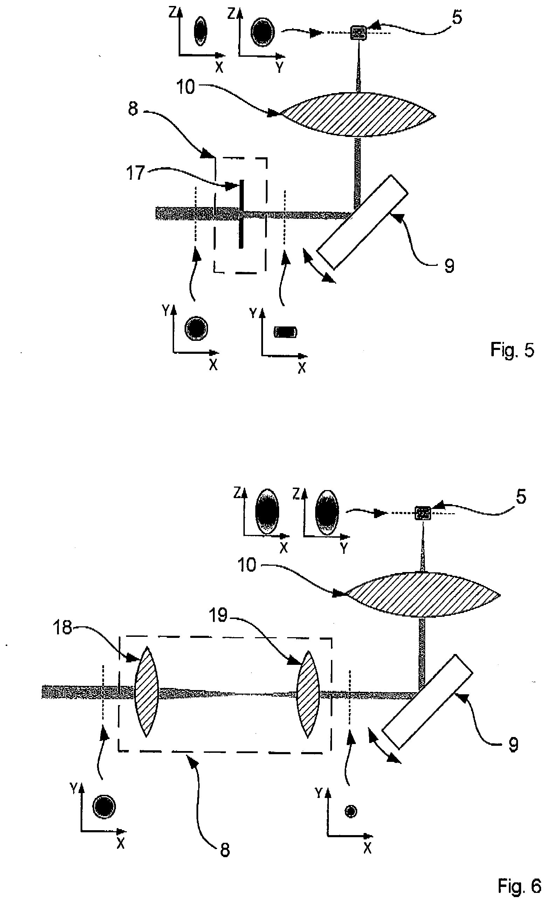

Ein ähnlicher Effekt ergibt sich bei Verwendung einer Schlitzblende 17, wie dies in der Ausbildung gemäß Fig. 5 gezeigt ist, wobei hier jedoch Intensitätsverluste durch das Abschneiden des Strahls entstehen.

Bei der Ausbildung gemäß Fig. 4 bewirkt eine Irisblende 16 eine Reduzierung der effektiven numerischen Apertur des Abbildungssystems, wodurch das Fokuspunktvolumen sowohl länger als auch breiter wird.

Der bei der Ausbildung gemäß Fig. 6 gezeigte Expander aus den Linsen 18 und 19 hat den gleichen Effekt wie die Irisblende 16, vermeidet aber Intensitätsverluste, indem der Strahldurchmesser verkleinert wird, ohne den Strahl abzuschneiden.

Die in den Fig. 2 bis 6 gezeigten Möglichkeiten zur Änderung des Fokuspunktvolumens erfolgen insbesondere unter Verwendung von gepulstem Laserlicht mit einer Wellenlänge im Bereich von 400 bis 1600nm, wobei die Pulslänge zwischen 1fs und Ins beträgt.

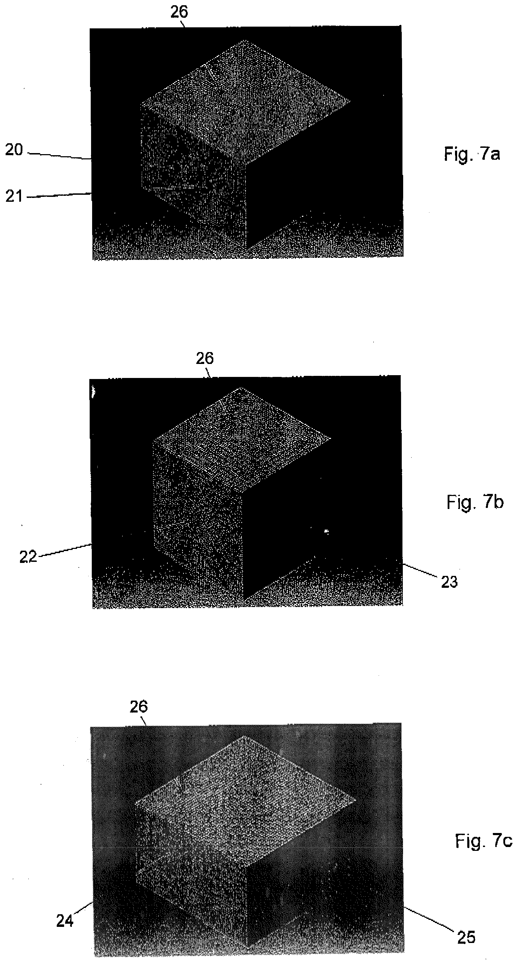

Fig. 7a - 7c zeigen nun mit einer geeigneten Software erstellte Modelle eines Nervengewebsphantoms mit unterschiedlichen Kanalstrukturen. In Fig. 7a ist exemplarisch ein Kanalpaar 20,21 ersichtlich, welches tangierend ausgebildet ist. In Fig. 7b sind einander kreuzende Kanalbündel 22, 23 ausgebildet, wobei die Kanäle der einzelnen Bündel 22,23 ineinandergreifend ausgebildet sind. Bei der Ausbildung gemäß Fig. 7c hingegen sind die Kanäle der einander kreuzenden Kanalbündel 24,25 hingegen in voneinander verschiedenen Abschnitten des Nervengewebsphantoms 26 ausgebildet.

In einem Versuch wurden die verschiedenen Nervengewebsphantome gemäß den Fig. 7a-c mit Hilfe eines Multiphotonenabsorptionsverfahren bzw. einem Multiphotonenlithographieverfahren gefertigt.

Beim einem würfelförmigen Nervengewebsphantom aus ETA-TTA mit einer Kantenlänge von 2,5 mm und dicht gepackten 10µm Kanälen konnte bereits eine leichte Erhöhung der Anisotropie ohne eindeutige Vorzugsrichtung innerhalb des Nervengewebsphantom gemessen werden. Im nächsten 7,0x7,0x1,5 mm³ großen Testdruck mit einem 200x40 Kanal-Array mit einem Kanalquerschnitt von 20x20µm konnte eine deutliche Erhöhung der Anisotropie in Kanalrichtung gemessen werden. Um Fehler in der Messung auszuschließen, wurde ein weiterer 6,0x6,0x2,5 mm³ großer Nervengewebsphantom mit einem Kanalbündel umfassend 121x40 Kanäle und einem senkrecht dazu verlaufenden Kanalbündel umfassend 121x40 Kanäle hergestellt und in Gelatine eingebettet. Diese Anordnung wurde dann aufgrund der nun ausreichenden Größe mit Hilfe eines 7 Tesla-Ganzkörperscanners gemessen. In den durchgeführten strukturellen Turbo-Spin-Echo-Messungen (TSE-Messungen) konnte der Aufbau der Phantome inklusive den einzelnen Druckbereichen genau rekonstruiert werden. Die Diffusions-Messungen mittels diffusionsgewichteten Echo-Planar-Imaging-Aufnahmen (EPI-Aufnahmen) zeigten eine deutliche Erhöhung der Anisotropie innerhalb der Nervengewebsphantomkanälee, sowie keine Anisotropie außerhalb der Nervengewebsphantomkanäle. Weiteres konnte nachgewiesen werden, dass die Anisotropie annähernd parallel zu den Kanälen verläuft.

## Patentansprüche

1. Verfahren zur Herstellung eines Nervengewebsphantoms, das eine Vielzahl von Kanälen in einem Strukturmaterial aufweist, wobei die Kanäle vorzugsweise einen maximalen Querschnitt von 625 µm² aufweisen, wobei ein virtuelles Modell des Nervengewebsphantoms erstellt und das virtuelle Modell einer Strukturierungseinrichtung zugeführt wird, welche das Nervengewebsphantom mit Hilfe eines 3D-Druck-basierten, insbesondere lithographiebasierten, Strukturierungsverfahrens herstellt, wobei das Strukturierungsverfahren als Multiphotonenlithographie-, insbesondere Multiphotonenabsorptionsverfahren ausgebildet ist, bei dem das einen Photosensibilisator oder Photoinitiator enthaltende Strukturmaterial ortsselektiv bestrahlt wird, wobei die Strahlung nacheinander auf Fokuspunkte innerhalb des Strukturmaterials fokussiert wird, wodurch jeweils ein im Fokuspunkt befindliches Volumenelement des Materials mittels einer photochemischen Reaktion in Folge von Multiphotonenabsorption eine Zustandsänderung erfährt.

2. Verfahren nach Anspruch 1, **dadurch gekennzeichnet, dass** das Strukturierungsverfahren als additives verfahren ausgebildet ist, bei dem das jeweils im Fokuspunkt befindliche Volumenelement des Materials verfestigt wird.

3. Verfahren nach Anspruch 1, **dadurch gekennzeichnet, dass** das Strukturierungsverfahren als subtraktives Verfahren ausgebildet ist, bei dem das jeweils im Fokuspunkt befindliche Volumenelement des Materials photodegradiert wird.

4. Verfahren nach Anspruch 1, 2 oder 3, **dadurch gekennzeichnet, dass** das Volumen des Fokuspunkts während des Verfahrens derart variiert wird, dass das Nervengewebsphantom zustandsveränderte, insbesondere verfestigte oder photodegradierte, Volumenelemente einer ersten Gruppe und zustandsveränderte, insbesondere verfestigte oder photodegradierte, Volumenelemente einer zweiten Gruppe umfasst, wobei die Volumenelemente der ersten Gruppe ein kleineres Volumen aufweisen als die Volumenelemente der zweiten Gruppe.

5. Verfahren nach Anspruch 4, **dadurch gekennzeichnet, dass** die erste Gruppe von verfestigten Volumenelementen die Kanalwände ausbilden und die zweite Gruppe von verfestigten Volumenelementen die zwischen den Kanalwänden liegenden Bereiche ausbilden.

6. Verfahren nach Anspruch 4, **dadurch gekennzeichnet, dass** die erste Gruppe von photodegradierten Volumenelementen die Kanalwände ausbilden und die zweite Gruppe von photodegradierten Volumenelementen den Innenraum der Kanäle ausbilden.

7. Verfahren nach Anspruch 4, 5 oder 6, **dadurch gekennzeichnet, dass** das Volumen des Fokuspunkts während des Verfahrens derart variiert wird, dass die zustandsveränderten Volumenelemente der ersten Gruppe ein Volumen aufweisen, das weniger als 50%, bevorzugt weniger als 10%, des Volumens der zustandsveränderten Volumenelemente der zweiten Gruppe beträgt.

8. Verfahren nach einem der Ansprüche 1 bis 7, **dadurch gekennzeichnet, dass** das Nervengewebsphantom eine erste Gruppe von Kanälen und eine zweite Gruppe von Kanälen umfasst, wobei die Kanäle der ersten Gruppe und die Kanäle der zweiten Gruppe nicht parallel zueinander verlaufen.

9. Verfahren nach Anspruch 8, **dadurch gekennzeichnet, dass** die Kanäle der ersten Gruppe die Kanäle der zweiten Gruppe kreuzen, vorzugsweise in einem rechten Winkel.

10. Verfahren nach Anspruch 8, **dadurch gekennzeichnet, dass** die Kanäle der ersten und der zweiten Gruppe gekrümmt ausgebildet sind und eine gemeinsame Tangente aufweisen.

11. Verfahren nach Anspruch 2, **dadurch gekennzeichnet, dass** als Strukturmaterial wenigstens ein Acrylat- oder Methacrylat basiertes Photopolymer, wie z.B. eine 1:1-Mischung von ethoxyliertem (20/3) - Trimethylolpropantriacrylat (ETA) und Trimethylolpropantriacrylat (TTA), vermischt mit einem Photoinitiator, eingesetzt wird.

12. Verfahren nach Anspruch 3, **dadurch gekennzeichnet, dass** der Photosensibilisator zum photochemischen Spalten von im Strukturmaterial enthaltenen photolabilen Bindungen, insbesondere Disulfidbindungen, ausgebildet ist, wobei der Photosensibilisator infolge der Zweiphotonen-Absorption, ohne eine intramolekulare Spaltung zu erfahren, die aufgenommene Energie auf die photolabilen Bindungen, insbesondere Disulfidbindungen, überträgt und dadurch deren Spaltung bewirkt.

13. Verfahren nach Anspruch 12, **dadurch gekennzeichnet, dass** das Strukturmaterial die zu spaltenden photolabilen Bindungen, insbesondere Disulfidbindungen, in einer von einem Polymernetzwerk, vorzugsweise einem über diese Bindungen vernetzten Polymernetzwerk, gebildeten Matrix enthält.

14. Verfahren nach Anspruch 13, **dadurch gekennzeichnet, dass** das Polymernetzwerk ein Gel, vorzugsweise ein Hydrogel, ist.

15. Verfahren nach Anspruch 14, **dadurch gekennzeichnet, dass** das Gel mit einer Lösung des Zweiphotonen-Photosensibilisators in einem geeigneten Lösungsmittel quellen gelassen wird, um den Zweiphotonen-Photosensibilisator im Gel zu verteilen, bevor die Bestrahlung erfolgt.

16. Verfahren nach einem der Ansprüche 12 bis 15, **dadurch gekennzeichnet, dass** eine Lösung des Zweiphotonen-Photosensibilisators mit einer Konzentration von etwa 0,01 mM bis etwa 1 mM, vorzugsweise von etwa 0,05 mM bis etwa 0,5 mM, insbesondere von etwa 0,1 mM, eingesetzt wird.

17. Verfahren nach einem der Ansprüche 12 bis 16, **dadurch gekennzeichnet, dass** ein aus der Klasse der Benzylidenketone ausgewählter Zweiphotonen-Photosensibilisator, vorzugsweise einer der nachstehenden dargestellten Initiatoren P2CK, G2CK und E2CK, eingesetzt wird:

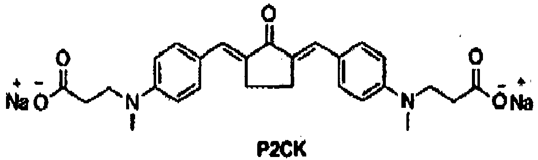

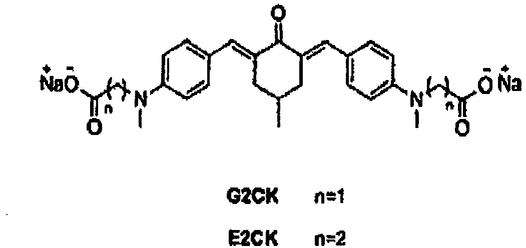

18. Nervengewebsphantom, hergestellt unter Verwendung eines Verfahrens nach einem der Ansprüche 1 bis 17.

19. Verwendung eines Nervengewebsphantoms nach Anspruch 18 zur Kalibrierung eines Magnetresonanztomographen, wobei das Nervengewebsphantom einer Diffusions-MRT unterworfen wird und die gewonnenen Diffusionsdaten einer quantitativen Analyse hinsichtlich der Diffusionskoeffizienten in den verschiedenen Richtungen unterworfen werden, wobei das Ergebnis der Diffusionskoeffizientenanalyse mit dem Referenzwert im Nervengewebsphantom verglichen wird und etwaige Abweichungen zur Kalibrierung des Magnetresonanztomographen herangezogen werden.

20. Verwendung eines Nervengewebsphantoms nach Anspruch 18 zur Kalibrierung eines Magnetresonanztomographen, wobei das Nervengewebsphantom einer Diffusions-MRT unterworfen wird und die gewonnenen Diffusionsdaten einer Traktographie unterworfen werden, wobei das Ergebnis der Traktographie mit dem Verlauf der Kanäle im Nervengewebsphantom verglichen wird und etwaige Abweichungen zur Kalibrierung des Magnetresonanztomographen herangezogen werden.
